Europäisches Patentamt

European Patent Office

Office européen des brevets

(19)

(11) Veröffentlichungsnummer: **0 246 523**
**B1**

(12) **EUROPÄISCHE PATENTSCHRIFT**

(45) Veröffentlichungstag der Patentschrift: **09.01.91**

(51) Int. Cl.⁵: **C 09 C 1/62, C 09 C 3/06**

(21) Anmeldenummer: **87106751.8**

(22) Anmeldetag: **09.05.87**

(54) **Perlglanzpigmente.**

(30) Priorität: **23.05.86 DE 3617430**

(43) Veröffentlichungstag der Anmeldung:
**25.11.87 Patentblatt 87/48**

(45) Bekanntmachung des Hinweises auf die
Patenterteilung:
**09.01.91 Patentblatt 91/02**

(84) Benannte Vertragsstaaten:
**BE CH DE ES FR GB IT LI NL**

(56) Entgegenhaltungen:
**DE-A-2 313 331
DE-A-3 534 477
FR-A-2 198 984
US-A-3 087 828
US-A-3 331 699
US-A-4 435 220**

**Die Akte enthält technische Angaben, die nach
dem Eingang der Anmeldung eingereicht
wurden und die nicht in dieser Patentschrift
enthalten sind.**

(73) Patentinhaber: **MERCK PATENT GESELLSCHAFT
MIT BESCHRÄNKTER HAFTUNG
Frankfurter Strasse 250 Postfach 4119
D-6100 Darmstadt (DE)**

(72) Erfinder: **Franz, Klaus-Dieter, Dr.
Insterburger Strasse 12
D-6233 Kelkheim (DE)**
Erfinder: **Ambrosius, Klaus, Dr.
Waldschmidtstrasse 65
D-6000 Frankfurt am Main (DE)**
Erfinder: **Knapp, August
Pater-Delp-Strasse 14
D-6110 Dieburg (DE)**
Erfinder: **Brückner, Hans-Dieter, Dr.
Behringstrasse 15
D-6100 Darmstadt 23 (DE)**

EP 0 246 523 B1

Courier Press, Leamington Spa, England.

**Beschreibung**

Die Anmeldung betrifft plättchenförmige farbige Pigmente, wobei ein plättchenförmiges Substrat mit einer eisenoxidhaltigen Schicht bedeckt ist.

Farbige eisenoxidhaltige plättchenförmige Pigmente sind an sich bekannt. Insbesondere werden dabei $Fe_2O_3$-Schichten, ggf. zusammen mit anderen Metalloxiden wie z.B. $TiO_2$, auf plättchenförmige Substrate, insbesondere Glimmer, aufgebracht. Darüberhinaus ist es jedoch auch aus der DE—OS 23 13 331 bekannt, eisen(II)-oxidhaltige Schichten abzuscheiden, wobei durch Fällung aus Eisen(II)-Salzlösungen in Gegenwart eines Oxidationsmittels einheitliche Schichten von Magnetit auf Glimmer oder auf mit Metalloxiden beschichteten Glimmer erzeugt werden. Wie im dritten Absatz auf Seite 7 der DE—OS 23 13 331 erläutert wird, sind dies rauhe Schichten, die keine Interferenzfarben und damit auch keinen Perlglanz aufweisen. Lediglich bei Verwendung von Glimmer/Metalloxid-Interferenzpigmenten als Substrat für eine Magnetitbeschichtung, kann bei sehr dünnen Magnetitschichten diese Interferenzfarbe noch durchscheinen, die Magnetitschicht selbst ist jedoch nicht in der Lage zur Erzeugung von Interferenzfarben dünner Plättchen.

Das gleiche trifft zu für die in Beispiel 8 der EP—A 00 77 959 beschriebenen schwarzen Pigmente, die analog dem Verfahren der DE—OS 23 13 331 hergestellt werden, wobei jedoch mit dem Ziel einer verbesserten Temperaturstabilität die Fällung des Magnetits in Gegenwart eines Erdalkalimetallsalzes vorgenommen wird.

Diese bekannten Magnetitpigmente sind zwar als plättchenförmige Schwarzpigmente zu vielen Zwecken gut einsetzbar, sind aber wegen fehlender Interferenzfähigkeit und mangelndem Glanz nicht als Perlglanzpigmente im eigentlichen Sinne anzusprechen.

Es bestand daher die Aufgabe, eisenoxidhaltige plättchenförmige Pigmente zu finden, die neben der Körperfarbe zusätzlich einen hohen Glanz aufweisen und auch in Abhängigkeit von der Schichtdicke die Interferenzfarbe dünner Plättchen zeigen können. Darüberhinaus sollten diese Pigmente sowohl für Anwendungen in der Technik als auch in der Kosmetik geeignet sein.

Diese Aufgabe wurde durch die vorliegende Erfindung gelöst. Es wurde nämlich gefunden, daß auf plättchenförmigen Substraten gleichmäßige glatte Schichten erzeugt werden können, die Eisen(II)-oxid enthalten, einen hohen Glanz aufweisen und dem Pigment eine attraktive Pulverfarbe verleihen, und die gegebenenfalls die Interferenzfarben dünner Plättchen erzeugen können.

Gegenstand der Anmeldung sind daher plättchenförmige farbige Pigmente, wobei ein plättchenförmiges Substrat mit einer aus Primärteilchen bestehenden eisenoxidhaltigen Schicht bedeckt ist, dadurch gekennzeichnet, daß die Schicht Eisen(II)-oxid enthält und daß die Primärteilchen eine Größe von 0,3 µm nicht überschreiten und so dicht gepackt sind, daß der Abstand zwischen benachbarten Teilchen in der Regel kleiner als deren Durchmesser ist.

Es wurde gefunden, daß diese interferenzfähigen eisenoxidhaltigen Schichten überraschenderweise sowohl naßchemisch durch direkte Ausfällung von Magnetit aus Eisen(II)-Salzlösungen in Gegenwart eines Oxidationsmittels hergestellt werden können als auch aus mit $Fe_2O_3$-beschichteten Pigmenten durch Erhitzen der Pigmente in Gegenwart eines reduzierenden Gases. Wesentlich bei der naßchemischen Herstellung ist es, daß im Gegensatz zu den bekannten Verfahren das Oxidationsmittel nicht in der Suspension des zu beschichtenden Substrats vorgelegt wird, sondern daß sowohl die Eisen(II)-Salzlösung als auch die Lösung des Oxidationsmittels simultan der Suspension zugegeben werden.

Gegenstand der Anmeldung ist daher auch ein Verfahren zur Herstellung der erfindungsgemäßen Pigmente, das dadurch gekennzeichnet ist, daß man zur Erzeugung von glänzenden Schichten, die die Interferenzfarben dünner Plättchen zeigen können, entweder eine wässerige Suspension eines plättchenförmigen Substrats bei weitgehend konstanter Temperatur und weitgehend konstantem pH-Wert gleichzeitig mit einer Eisen(II)-Salzlösung und der Lösung eines Oxidationsmittels versetzt und das Pigment abtrennt und gegebenenfalls wäscht und trocknet oder ein auf an sich bekannte Weise mit einer glatten homogenen $Fe_2O_3$-Schicht überzogenes plättchenförmiges Substrat bei einer Temperatur oberhalb 100°C einer reduzierenden Atmosphäre aussetzt.

Gegenstand der Anmeldung ist weiterhin die Verwendung dieser Pigmente zur Pigmentierung von Lacken, Farben, Kunststoffen und Kosmetika.

Im Rahmen der vorliegenden Erfindung werden erstmalig eisen(II)-oxidhaltige plättchenförmige Pigmente zugänglich gemacht, die über einen hohen Glanz und die Interferenzfarben dünner Plättchen verfügen. Entscheidend dafür ist, daß die eisenoxidhaltige Schicht der neuen Pigmente aus sehr viel kleineren und dichter gepackten Primärteilchen besteht als die der bekannten Pigmente. Wie aus rasterelektronenmikroskopischen (REM) Aufnahmen ersichtlich ist, liegen bei den bekannten, beispielsweise nach der DE—OS 23 13 331 oder der EP—OS 00 77 959 hergestellten Pigmenten die Magnetitprimärteilchen als relativ grobe Kristalle von etwa 0,5 µm Durchmesser vor, die unregelmäßig und mit relativ großen freien Zwischenräumen auf der Oberfläche des Substrats verteilt sind.

Bei den erfindungsgemäßen Pigmenten dagegen sind die Primärteilchen deutlich kleiner und liegen vor allem auch in dicht gepackter Verteilung auf dem Substrat vor.

Die Größe der Primärteilchen ist im wesentlichen kleiner als 0,3 µm und je nach dem angewandten Herstellungsverfahren sogar zum Teil deutlich unterhalb 0,1 µm. Entscheidend ist jedoch, daß in jedem Fall die Packung der Teilchen so dicht ist, daß die Zwischenräume zwischen benachbarten Primärteilchen in der Regel geringer als der Teilchendurchmesser sind.

Das Verfahren zur Herstellung dieser vorteilhaften neuen Pigmente ist ebenfalls neu. Man kann dabei entweder direkt naßchemisch eine Magnetitschicht auf ein geeignetes plättchenförmiges Substrat auffällen oder aber das plättchenförmige Substrat zunächst mit Eisen(III)oxid beschichten, das anschließend zu einer eisen(II)-oxid-haltigen Schicht reduziert wird.

Als plättchenförmiges Substrat kommen an sich alle plättchenförmigen Materialien in Frage, die unter den Beschichtungsbedingungen stabil sind, wie z.B. Glimmer, Glasplättchen, Metallplättchen, Graphit und andere plättchenförmige Materialien.

Bevorzugt wird Glimmer, wie z.B. Muskovit oder Phlogopit eingesetzt. Als plättchenförmiges Substrat können jedoch auch Materialien eingesetzt werden, die bereits eine Metalloxidbeschichtung besitzen, insbesondere Glimmerplättchen mit einer oder mehreren Beschichtungen aus z.B. $TiO_2$, $ZrO_2$, $SnO_2$, $Cr_2O_3$, BiOCl, $Fe_2O_3$, $Al_2O_3$, $SiO_2$, ZnO oder Gemischen dieser Metalloxide.

Die Größe dieser plättchenförmigen Substrate ist an sich nicht kritisch und es können daher Teilchen in der für die beabsichtigte Anwendung geeigneten Größe verwendet werden. In der Regel wird man das Substrat in Teilchengrößen von etwa 1 bis 200 µm, insbesondere etwa 5 bis 100 µm einsetzen. Die Dicke der Teilchen liegt in der Regel bei etwa 0,1 bis 5 µm, insbesondere etwa 0,5 µm.

Die als Substrate verwendeten Ausgangsmaterialien sind bekannt oder lassen sich nach bekannten Verfahren herstellen. Glimmerteilchen der gewünschten Größenordnung lassen sich durch Vermahlen von Glimmer und nachfolgendes Klassieren gewinnen. Metalloxidbeschichtete Materialien, insbesondere metalloxidbeschichtete Glimmerplättchen, sind sowohl käuflich erhältlich z.B. von E. Merck, Darmstadt, als Iriodin[(R)]-Perlglanzpigmente oder sind nach bekannten Verfahren herstellbar. Solche Verfahren sind z.B. beschrieben in den Patenten bzw. Patentanmeldungen US 30 87 828, US 30 87 829, DE 19 59 998, DE 20 09 566, DE 22 14 545, DE 22 44 298, DE 23 13 331, DE 25 22 572, DE 31 37 808, DE 31 37 809, DE 31 51 343, DE 31 51 354, DE 31 51 355, DE 32 11 602 und DE 32 35 017.

Je nach dem Herstellungsverfahren und dem verwendeten Substrat kann die Eisen(II)-oxid-haltige Schicht eine unterschiedliche Zusammensetzung haben. So ist es möglich, auf naßchemischem Wege eine Magnetitbeschichtung aufzubringen. Im Gegensatz zu den bekannten Verfahren erhält man jedoch nach dem erfindungsgemäßem Verfahren kompakte glänzende Schichten, die bei ausreichender Schichtdicke auch die Interferenzfarbe dünner Plättchen zeigen.

Wenn als Substrat metalloxidbeschichtete Plättchen eingesetzt werden, kann es an der Phasengrenze Magnetit/Metalloxid zur Ausbildung von Mischphasen kommen. Auch diese Mischphasen sind als Magnetit-Schichten im Sinne der Erfindung zu verstehen.

Die eisen(II)-oxidhaltige Schicht kann jedoch auch als weitgehend reine Eisen(II)oxid-Schicht vorliegen (Wüstit-Phase; $Fe_{0,90-0,95}O$) oder als Mischoxid mit anderen Metalloxiden. Als solche Mischoxide können z.B. genannt werden Eisenaluminat ($FeAl_2O_4$), Chromeisenstein ($FeCr_2O_4$), Eisenorthosilicat ($FeSiO_4$) und insbesondere auch Ilmenit ($FeTiO_3$).

Sowohl Wüstit als auch die Mischoxide mit anderen Metallen werden insbesondere bei der Reduktion von Eisen(III)oxid-haltigen Schichten mit einem reduzierenden Gas bei erhöhter Temperatur erhalten. Auch hier kann es in Abhängigkeit vom verwendeten Substrat insbesondere an den Phasengrenzen zur Ausbildung weiterer Mischphasen kommen. Auch diese sind in der erfindungsgemäßen Definition von Eisen(II)oxidhaltigen Schichten erfaßt.

Zur naßchemischen Beschichtung mit Magnetit werden die Substrate in Wasser suspendiert und es wird bei geeigneter Temperatur und geeignetem pH-Wert eine Eisen(II)-Salzlösung und ein Oxidationsmittel zugegeben. Der geeignete Temperaturbereich liegt bei etwa 0 bis 100°C; vorzugsweise arbeitet man bei etwa 50 bis 100°C. Der pH-Wert der Suspension sollte oberhalb 7 liegen; vorzugsweise stellt man einen pH-Wert zwischen 8 und 11 ein.

Die Zugabe des Eisen(II)-Salzes, wie z.B. Ammonium-Eisen(II)-Sulfat, Eisen(II)-Halogenide oder insbesondere Eisen(II)-Sulfat erfolgt so, daß das ausgefällte Eisenoxidhydrat unmittelbar auf dem Substrat niedergeschlagen wird und es zu keinen Nebenfällungen in der Suspension kommt. Während der Fällung wird der pH-Wert möglichst weitgehend konstant gehalten. Am zweckmäßigsten geschieht dies durch gleichzeitige Zugabe einer Base, wie z.B. NaOH, KOH oder Ammoniak; es ist jedoch auch möglich ein geeignetes Puffersystem zu verwenden.

Die Fällung des Eisenoxids wird in Gegenwart eines Oxidationsmittels, vorzugsweise eines Nitrats oder auch eines Chlorats, vorgenommen, das möglichst in stöchiometrischer Menge eingesetzt wird, d.h. bei Verwendung von Nitrat 1 Mol des Nitrats für maximal 12 Mol Eisen(II)-Ionen. Entscheidend ist, daß das Oxidationsmittel nicht wie bei den Verfahren des Standes der Technik in der Suspension vorgelegt, sondern gleichzeitig mit der Eisen(II)-Salzlösung der Suspension in der benötigten stöchiometrischen Menge zudosiert wird. Überraschenderweise werden dabei sehr glatte dichte Schichten von $Fe_3O_4$ abgeschieden, die im Gegensatz zu denen des Standes der Technik die Interferenzfarben dünner Plättchen erzeugen. In REM-Aufnahmen ist deutlich erkennbar, daß die Magnetitschicht aus feinkristallinen dicht gepackten Kristallen mit einer Größe im Bereich von etwa 0,1 bis 0,3 µm besteht.

Die eisenoxidhaltige Schicht kann je nach dem gewünschten Effekt Dicken bis zu etwa 500 nm, vorzugsweise 0,1 bis 250 nm, aufweisen. Dabei werden in der Regel Gehalte an $Fe_3O_4$ erreicht, die bezogen auf das Substrat etwa 0,1 bis 200 Gew.%, insbesondere etwa 5 bis 100 Gew.% ausmachen. Je nach der Schichtdicke der Magnetitschicht werden dabei Interferenzfarben erzielt, die mit wachsender Schichtdicke von silber über gold, rot, violett und blau bis hin zu grün und schließlich Interferenzfarben höherer Ordnung gehen.

Bei Erreichen der gewünschten Interferenzfarbe wird die Beschichtung abgebrochen und das beschichtete Substrat wird in der Regel aus dem Reaktionsgemisch abgetrennt, mit Wasser gewaschen und getrocknet. Zur Vermeidung von unerwünschter Oxidation kann die Trocknung gegebenenfalls in einer Inertgasatmosphäre wie z.B. Stickstoff oder gar unter Zumischung einer reduzierenden Gases wie z.B. Wasserstoff vorgenommen werden. In der Regel wird bei Temperaturen von etwa 80 bis 120°C getrocknet, insbesondere in einer $N_2/H_2$-Atmosphäre können jedoch auch höhere Temperaturen von bis zu 800°C angewendet werden, wobei eine zusätzliche Sinterung der Magnetitschicht auftritt.

In manchen Fällen ist es zweckmäßig, zusätzlich eine Deckschicht auf den neuen Pigmenten vorzusehen. Zweckmäßig wird man hierfür in bekannter Weise Schichten farbloser Oxide wählen, z.B. Titandioxid, Zirkondioxid, Aluminiumoxid, Antimonoxid, Zinkoxid, Siliziumdioxid, Magnesiumoxid oder Zinndioxid, die jeweils allein oder auch im Gemisch aufgebracht werden können. Eine solche Deckschicht kann nach den üblichen Methoden auf bereits getrocknete Pigmente aufgebracht werden oder auch noch einfacher vor der Abtrennung der Pigmente aus der Fälllösung. Im allgemeinen ist die Deckschicht dünner als die erfindungsgemäß aufgebrachten Schichten. Eine Schicht aus Aluminiumoxidhydrat oder Aluminiumoxid bewirkt meist eine zusätzliche Stabilisierung, und zwar sowohl in Bezug auf mechanische Eigenschaften als auch im Hinblick auf Witterungsbeständigkeit. Die Schichtdicke ist hier nicht besonders kritisch, da Aluminiumoxidhydrate und -oxide einen verhältnismäßig niedrigen Brechungsindex besitzen. Die Methoden zur Aufbringung solcher Schichten sind bekannt und z.B. in der deutschen Offenlegungsschrift 14 67 468 beschrieben. Zur Verbesserung der Temperaturstabilität der Pigmente kann die Fällung des Magnetits auch in Gegenwart eines Erdalkalimetallsalzes entsprechend der EP—OS 77 959 vorgenommen werden.

Alternativ zum naßchemischen Verfahren kann eine erfindungsgemäße eisen(II)oxidhaltige Beschichtung jedoch auch durch Reduktion einer zuvor aufgebrachten $Fe_2O_3$-haltigen Schicht erfolgen. Als Ausgangsmaterial kommen dabei alle obengenannten Substrate in Frage. Diese plättchenförmigen Materialien können auf bekannte Weise mit Eisenoxid bzw. -oxidhydrat beschichtet werden. Solche Verfahren sind beschrieben z.B. in der US—PS 30 87 828, US—PS 30 87 929, DE—OS 19 59 998, DE—OS 22 44 298, DE—OS 23 13 331, DE—OS 27 23 871, DE—OS 30 30 056 und der DE—OS 32 37 264. Eisenoxidbeschichtete Pigmente auf Glimmerbasis sind auch käuflich erhältlich. Dabei sind insbesondere die von der Firma E. Merck, Darmstadt, vertriebenen Pigmente mit der Warenbezeichnung Iriodin[(R)] 400, 500, 502, 504, 520, 522, 524 und 530 sowie die von der Firma Mearl, USA, vertriebenen Typen Mearl-Russet, Cloisonne-Russet, Bronze und Copper zu nennen.

Die entweder nach einem der bekannten Verfahren mit Eisenoxid bzw. -oxidhydrat beschichteten Substrate oder die käuflich erhältlichen Pigmente werden dann bei erhöhter Temperatur oberhalb 100°C einer reduzierenden Atmosphäre ausgesetzt. Insbesondere werden dabei Temperaturen von etwa 200 bis 1000°C, vorzugsweise von 400 bis 800°C angewendet. Als Reduktionsmittel kommen im Prinzip alle reduzierenden Gase in Betracht. Beispielhaft werden genannt Wasserstoff, Kohlenmonoxid, Methan und Ammoniak, wobei Wasserstoff bevorzugt eingesetzt wird. Diese Gase können in reiner Form oder aber auch verdünnt mit einem Inertgas wie z.B. Stickstoff, Argon, Helium oder auch Wasserdampf eingesetzt werden. Bevorzugt verwendet man Mischungen, die etwa 20 bis 60% das reduzierenden Gases enthalten.

Die Umwandlung von $Fe_2O_3$ in Eisen(II)oxid, Magnetit oder Mischphasen von Eisen(II)oxid mit anderen Metalloxiden erfolgt je nach Temperatur und der Art des reduzierenden Gases oder Gasgemisches mehr oder weniger schnell. Für die Zeitdauer der Reduktion ist auch entscheidend, wie dick die zu reduzierende $Fe_2O_3$-haltige Schicht ist und ob man die gesamte Schicht umwandeln möchte oder nur eine mehr oder weniger dicke Deckschicht. Der Zeitraum kann also in sehr breitem Rahmen variieren. In jedem Fall kann jedoch durch einige orientierende Versuche die optimale Reduktionszeit vermittelt werden. In der Regel werden Zeiträume von etwa 0,25 bis 2 Stunden angemessen sein. Die Reduktion kann im Prinzip in jedem mit reduzierendem Gas zu beschickenden Ofen durchgeführt werden. Um kontinuierlich arbeiten zu können wird bevorzugt ein Drehrohrofen eingesetzt. Auch die Art der erzeugten Eisen(II) oxid-haltigen Schicht kann durch die Temperatur beeinflußt werden. So werden reine $Fe_2O_3$-Schichten bei relativ niedriger Temperatur wie z.B. etwa 400—500°C vorwiegend in Magnetit umgewandelt, während bei hohen Temperaturen von etwa 700—900°C die Wüstit-Phase gebildet wird.

Bei der thermischen Behandlung mit einem reduzierenden Gas wird die Qualität der durch Reduktion erzeugten Schicht in erster Linie durch die Qualität der ursprünglichen $Fe_2O_3$-haltigen Schicht bestimmt. Da es möglich ist, sehr feinkristalline homogene und dicht gepackte $Fe_2O_3$-haltige Beschichtungen herzustellen, ist es auf diesem Wege möglich, Eisen(II)oxid-haltige Schichten der gleichen Qualität zu gewinnen. So werden z.B. bei der Reduktion der im Handel erhältlichen Iriodin[(R)]-Pigmente der obenerwähnten 500-er Reihe Pigmente erhalten, deren Untersuchung im REM erkennen läßt, daß die Primärteilchen in der Schicht eine Größe von nur etwa 0,1 µm und darunter aufweisen. Die Reduktion kann auch mit den ungeglühten $Fe_2O_3$-haltigen Präparaten erfolgen. Die durch Reduktion von kompakten $Fe_2O_3$-Schichten gewonnenen eisen(II)oxid-haltigen Schichten sind daher besonders glänzend und stabil.

Die neuen Pigmente stellen eine wesentliche Bereicherung der Technik dar. Durch die dunkle, bis zum schwarz gehende Pulverfarbe und die beliebig erzeugbare Interferenzfarbe, ergeben sich äußerst interessante Effekte, die zu vielfältigen Anwendungen ausgenutzt werden können, wobei insbesondere bei Substraten, die bereits selbst eine Interferenzfarbe aufweisen, diese beispielsweise durch eine Eisen(II)-oxid-Titandioxid-Mischphase (Ilmenit) oder eine Magnetit-Schicht verstärkt und variiert werden kann. Anwendungsgebiete ergeben sich sowohl in der Kosmetik, wo die erfindungsgemäßen Pigmente z.B. in Pudern, Salben, Emulsionen, Fettstiften und anderen Mitteln in Konzentrationen von in der Regel zwischen 0,1 und 80% eingesetzt werden, als auch in der Technik, z.B. zur Pigmentierung von Farben, Lakken oder Kunststoffen. Der Vorteil der erfindungsgemäßen Pigmente bei der Anwendung in der Kosmetik besteht einmal darin, daß beispielsweise Magnetit als Kosmetikpigment zugelassen ist und daß zum anderen mit einem einzigen Pigment sowohl ein hervorragender Farbglanz als auch eine schwarze Körperfarbe geliefert werden kann.

Darüberhinaus gibt es noch weitere Anwendungsgebiete für plättchenförmige Eisenoxide von der Kristallstruktur des Magnetits, in denen die Kombination ihrer elektromagnetischen Eigenschaften mit der Form ausgenutzt wird. Die magnetischen Wechselwirkungen führen zu einer stark ausgeprägten parallelen Orientierung der Einzelteilchen in Beschichtungsmaterialien. Im Vergleich zu herkömmlichen Eisenoxiden lassen sich folglich sehr viel höhere Packungsdichten erreichen, was sich beispielsweise in einer erhöhten korrosionsschützenden Wirkung, in einer guten Abschirmung gegen elektromagnetische Störfelder, sowie in einer hohen Leitfähigkeit äußert.

Die leichte Ausrichtbarkeit plättchenförmiger, magnetischer Teilchen in Magnetfeldern und ihr je nach Orientierung der Plättchen relativ zu einem einfallenden Lichtstrahl unterschiedliches Lichtstreuvermögen kann man sich für magneto-optische Anzeigen zunutze machen. Die Ausnutzung des Faraday-Effektes eröffnet für die erfindungsgemäßen plättchenförmigen Eisenoxide die Verwendung für magneto-optische Speicher.

## Beispiel 1

Zu einer Suspension von 100 g Kaliglimmer vom Durchmesser 5 bis 50 µm in 2500 ml Wasser werden bei 80°C und einem pH-Wert von 8 unter kräftigem Rühren innerhalb einer Stunde gleichzeitig eine Lösung von 600 g $FeSO_4 \cdot 7 H_2O$ in 2000 ml Wasser, die mit 50 ml konzentrierter Schwefelsäure angesäuert ist, sowie eine Lösung von 150 g $KNO_3$ in 2000 ml Wasser zudosiert, wobei der pH-Wert durch Zugabe von 15%iger Natronlauge konstant gehalten wird. Danach wird das blauschwarz glänzende Pigment mit Magnetitbeschichtung abfiltriert, gewaschen und 3 Stunden bei 100°C getrocknet.

## Beispiel 2

Es wird analog Beispiel 1 gearbeit, wobei jedoch eine Lösung von 350 g $FeSO_4 \cdot 7 H_2O$ und eine Lösung von 90 g $KNO_3$ zudosiert wird. Man erhält ein schwarzgoldenes Pigment mit Magnetitbeschichtung.

## Beispiel 3

Es wird analog Beispiel 1 gearbeitet, wobei jedoch eine Lösung von 450 g $FeSO_4 \cdot 7 H_2O$ und eine Lösung von 112 g $KNO_3$ zudosiert wird. Man erhält ein schwarzrotes Pigment mit Magnetitbeschichtung.

## Beispiel 4

Es wird analog Beispiel 1 gearbeitet, wobei jedoch eine Lösung von 700 g $FeSO_4 \cdot 7 H_2O$ und eine Lösung von 175 g $KNO_3$ zudosiert wird. Man erhält ein schwarzgrünes Pigment mit Magnetitbeschichtung.

## Beispiel 5

Es wird analog Beispiel 1 gearbeitet, wobei jedoch als Ausgangsmaterial ein Glimmer/$TiO_2$-Pigment mit roter Interferenzfarbe, entsprechend Beispiel 4 der DE—PS 14 67 468 eingesetzt wird, das unter Verwendung von 250 g $FeSO_4 \cdot 7 H_2O$ und 50 g $KNO_3$ zu einem schwarzgrünen Pigment beschichtet wird.

## Beispiel 6

50 g eines rotbraun glänzenden Glimmer/$Fe_2O_3$-Pigmentes mit einem Eisenoxidgehalt von 43 Gew.% (hergestellt nach Beispiel 1 b der DE—OS 23 13 331) werden bei 800°C für 0,5 Stunden in einem 50 cm langen Durchflußrohr mit einem 1:1 Gemisch aus Stickstoff und Wasserstoff bei 100 l pro Stunde Durchflußgeschwindigkeit umgesetzt. Man erhält ein anthrazitglänzendes plättchenförmiges Produkt, das im sofort aufgenommenen Debye-Scherrer-Diagramm die charakteristischen Linien von FeO und Glimmer aufweist.

## Beispiel 7

Es wird analog Beispiel 6 gearbeitet, wobei jedoch anstelle des kalzinierten $Fe_2O_3$-Glimmerpigmentes die lediglich getrocknete Vorstufe eingesetzt wird. Bei einer Reduktionstemperatur von 500°C erhält man ein schwarzglänzendes Produkt mit roter Interferenzfarbe, das im Debye-Scherrer-Diagramm die charakteristischen Linien von $Fe_3O_4$ und Glimmer zeigt.

## Beispiel 8

Im gleichen Durchflußrohr wie in Beispiel 6 werden 66 g eines Pigmentes, das eine Beschichtung von 6 g $Fe_2O_3$ in ungeglühter Form auf 60 g

eines geglühten TiO$_2$-Glimmerpigments mit blauer Interferenzfarbe aufweist, bei 800°C reduziert. Man erhält ein tief dunkelblaues metallisch glänzendes Pigment, das im Debye-Scherrer-Diagramm die charakteristischen Linien von Ilmenit (FeTiO$_3$)$_2$, TiO$_2$ und Glimmer aufweist.

## Patentansprüche

1. Plättchenförmige farbige Pigmente, wobei ein plättchenförmiges Substrat mit einer aus Primärteilchen bestehenden eisenoxidhaltigen Schicht bedeckt ist, dadurch gekennzeichnet, daß die Schicht Eisen(II)-oxid enthält und daß die Primärteilchen ein Größe von 0,3 μm nicht überschreiten und so dicht gepackt sind, daß der Abstand zwischen benachbarten Teilchen in der Regel kleiner als deren Durchmesser ist.

2. Pigment nach Anspruch 1, dadurch gekennzeichnet, daß das Eisen(II)-oxid als Mischoxid mit den Oxiden TiO$_2$, ZrO$_2$, SnO$_2$, Cr$_2$O$_3$, BiOCl, Fe$_2$O$_3$, Al$_2$O$_3$, SiO$_2$, ZnO oder mit Gemischen davon vorliegt.

3. Verfahren zur Herstellung von Pigmenten nach Anspruch 1, dadurch gekennzeichnet, daß man zur Erzeugung von glänzenden Schichten, die die Interferenzfarben dünner Plättchen zeigen können, entweder eine wässerige Suspension eines plättchenformigen Substrats bei weitgehend konstanter Temperatur und weitgehend konstantem pH-Wert gleichzeitig mit einer Eisen(II)-Salzlösung und der Lösung eines Oxidationsmittels versetzt und das Pigment abtrennt und gegebenenfalls wäscht und trocknet oder ein auf an sich bekannte Weise mit einer glatten homogenen Fe$_2$O$_3$-Schicht überzogenes plättchenförmiges Substrat bei einer Temperatur oberhalb 100°C einer reduzierenden Atmosphäre aussetzt.

4. Verwendung der plättchenförmigen Pigmente nach Anspruch 1 oder 2 zur Pigmentierung von Lacken, Farben, Kunststoffen oder Kosmetika.

## Revendications

1. Pigments lamellaires colorés, dans lesquels un substrat lamellaire est revètu d'une couche renfermant de l'oxyde de fer constituée de particules primaires, caractérisé en ce que la couche comporte de l'oxyde ferreux et en ce que la grosseur des particules primaires n'excède pas 0,3 μm et que ces particules sont entassées de manière telle que la distance séparant des particules voisines est généralement inférieure à leur diamètre.

2. Pigment selon la revendication 1, caractérisé en ce que l'oxyde ferreux est présent comme oxyde mixte avec les oxydes TiO$_2$, ZrO$_2$, SnO$_2$, Cr$_2$O$_3$, BiOCl, Fe$_2$O$_3$, Al$_2$O$_3$, SiO$_2$, ZnO ou avec des mélanges de ceux-ci.

3. Procédé de fabrication des pigments selon la revendication 1, caractérisé en ce que les couches lustrées qui peuvent montrer les couleurs d'interférence de minces lamelles sont obtenues soit par mélange d'une suspension aqueuse d'un substrat lamellaire à température et à pH largement constants, simultanément avec une solution de sel ferreux et la solution d'agent d'oxydation, le pigment étant ensuite séparé et, éventuellement, lavé et séché, soit par exposition à une atmosphère réductrice d'un substrat lamellaire revêtu par un procédé connu en soi d'une couche de Fe$_2$O$_3$ lisse et homogène, à une température supérieure à 100°C.

4. Utilisation des pigments lamellaires selon les revendications 1 ou 2 pour la pigmentation de vernis, peintures, matières plastiques ou cosmétiques.

## Claims

1. Platelet-shaped coloured pigments in which a platelet-shaped substrate is covered with a layer consisting of primary particles and containing iron oxide, characterized in that the layer contains iron(II) oxide and in that the primary particles do not exceed a size of 0.3 μm and are so densely packed that the distance between adjacent particles is as a rule smaller than the diameter thereof.

2. Pigment according to Claim 1, characterized in that the iron(II) oxide is present as a mixed oxide with the oxides TiO$_2$, ZrO$_2$, SnO$_2$, Cr$_2$O$_3$, BiOCl, Fe$_2$O$_3$, Al$_2$O$_3$, SiO$_2$, ZnO or with mixtures thereof.

3. Process for the preparation of pigments according to Claim 1, characterized in that, to produce lustrous layers which can exhibit the interference colours of thin platelets, either an iron(II) salt solution and the solution of an oxidizing agent are simultaneously added to an aqueous suspension of a platelet-shaped substrate at a largely constant temperature and largely constant pH and the pigment is separated off and, if appropriate, washed and dried, or a platelet-shaped substrate coated with a smooth homogeneous layer of Fe$_2$O$_3$ in a manner which is known per se is exposed to a reducing atmosphere at a temperature above 100°C.

4. Use of the platelet-shaped pigments according to one of Claim 1 or 2 for pigmenting lacquers, paints, plastics or cosmetics.